# FASCICULE DE BREVET EUROPEEN

(11) **EP 0 983 774 B1**
(45) Date de publication et mention de la délivrance du brevet: **03.11.2004**
(21) Numéro de dépôt: 99402154.1
(22) Date de dépôt: 31.08.1999
(51) Int. Cl.: A61M 25/06

(54) **Embase d'aiguille de ponction artérielle**
Nadelhalterung für Arterienpunktion
Needle hub for artery tapping

(30) Priorité: 01.09.1998 FR 9810904
(43) Date de publication de la demande: 08.03.2000
(73) Titulaire: VYGON, F-95440 Ecouen (FR)
(72) Inventeur: Huet, Jean-Max, 92110 Clichy (FR); Rossi, Daniel, 95630 Meriel (FR); Brinon, Thierry, 95560 Montsoult (FR)
(74) Mandataire: Schrimpf, Robert

(56) Documents cités:
- EP-A- 0 812 601
- EP-A- 0 812 602
- US-A- 5 439 449

## Description

L'invention concerne une embase d'aiguille de ponction artérielle.

Ces aiguilles sont utilisées notamment pour permettre l'introduction d'un cathéter dans l'artère, ce cathéter étant enfoncé dans l'artère en coulissant sur l'aiguille qui le guide (méthode dite du cathéter court) ou en coulissant sur un guide métallique introduit préalablement dans l'artère via l'aiguille de ponction (méthode dite de Seldinger).

Le praticien sait que l'aiguille est bien en place dans l'artère par l'observation du sang qui reflue dans l'aiguille.

Pour l'observation de ce reflux sanguin sans risque de contamination du praticien, il est connu dans le cas d'un cathéter court d'utiliser une aiguille dont l'embase comporte une chambre de visualisation tubulaire transparente accessible par une extrémité d'entrée au reflux de sang dans l'aiguille et fermée à une extrémité opposée de sortie par un bouchon étanche au sang mais perméable à l'air.

La pression artérielle, supérieure à la pression atmosphérique, fait que dès la pénétration de l'aiguille dans l'artère, le reflux de sang remplit totalement la chambre.

Si la ponction doit être répétée, soit le praticien enlève le bouchon pour vider la chambre et remet le bouchon en place mais il ne peut contrôler la nouvelle introduction de l'aiguille dans l'artère car les parois de la chambre de visualisation sont déjà rouges du sang de la ponction précédente, soit il laisse l'extrémité proximale de l'aiguille ouverte, le sang s'échappant librement de l'embase.

La présente invention vise à permettre la visualisation de la pression sanguine même dans ce cas et, plus généralement, pendant toute la durée de l'opération d'introduction du cathéter dans l'artère sans manipulation ni contact avec le sang du patient.

On a proposé de munir l'embase de moyens pour créer une contre-pression suffisante pour maintenir à l'extrémité de sortie de la chambre de visualisation une colonne d'air dont le volume varie sous l'effet des variations de la pression du sang dans l'aiguille, ces moyens étant constitués par une chambre de réserve d'air étanche vis à vis de l'air ambiant, située à l'extérieur de la chambre de visualisation et en communication d'air avec l'extrémité de sortie de la chambre de visualisation (US 5 439 449). Dans cette solution connue, la chambre de réserve d'air est constituée par un réservoir élastomère relié par une liaison souple à l'embase et protégé par un carter.

La présente invention vise à fournir une solution simple telle que l'ensemble du dispositif soit facile à prendre en main sans appendice souple qui peut être gênant pour la ponction, et d'un fonctionnement sûr.

On y parvient selon l'invention en utilisant une chambre de visualisation qui forme avec l'embase et la chambre de réserve d'air un ensemble rigide, les volumes des chambres étant prédéterminés pour que le sang soit visible dans la chambre de visualisation lorsque la pression diastolique est minimale et qu'il n'atteigne pas la chambre de réserve d'air lorsque la pression systolique est maximale.

Lorsque l'aiguille pénètre dans l'artère, le sang reflue dans l'aiguille et ne remplit que partiellement la chambre de visualisation, l'air contenu initialement étant comprimé à l'intérieur de la chambre de réserve d'air et la variation de pression artérielle mettant le sang en mouvement à l'intérieur de la chambre de visualisation.

Pour rendre le dispositif plus compact, il est avantageux, selon une particularité de l'invention, de placer la réserve d'air autour de la chambre de visualisation.

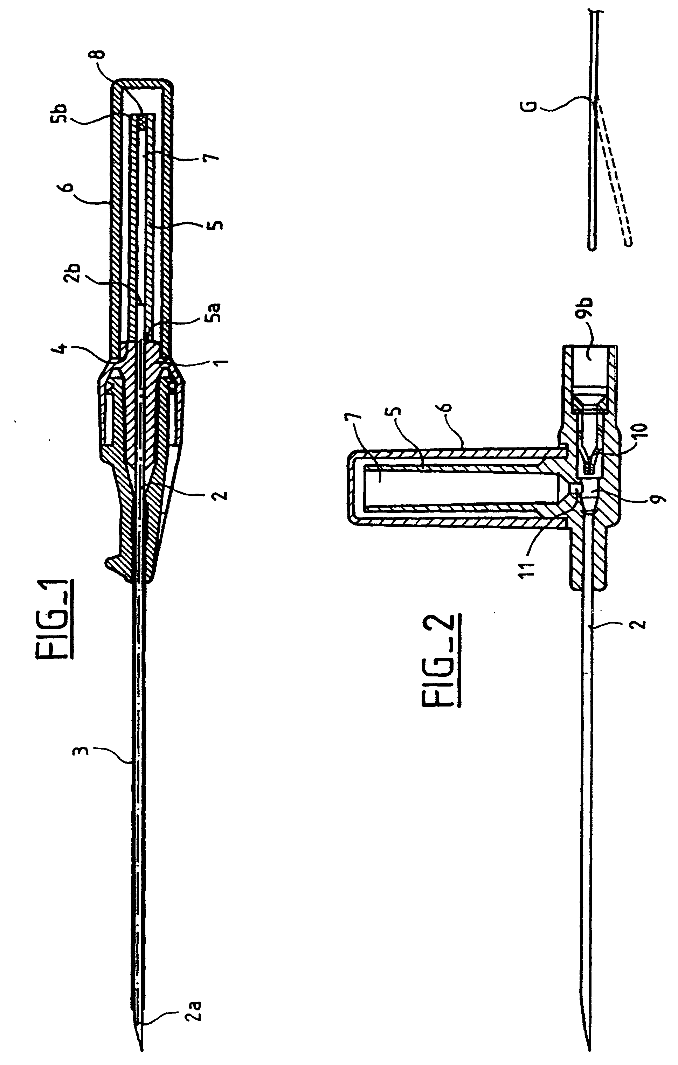

On décrira ci-après deux modes de réalisation d'une embase d'aiguille conforme à la présente invention, en référence aux figures du dessin joint, sur lesquelles la figure 1 est une coupe longitudinale d'une embase de cathéter court, et la figure 2 est un schéma d'une embase d'aiguille de ponction utilisée pour la mise en place d'un cathéter par la méthode de Seldinger.

L'embase d'aiguille du cathéter représentée sur la figure 1 est un corps creux tubulaire (1) en matière synthétique traversé par une aiguille (2) dont l'extrémité distale (2a) est biseautée.

Sur l'aiguille est enfilé un tube cathéter (3). A son extrémité proximale (4) l'embase (1) est prolongée par un tube de visualisation (5) qui présente une extrémité d'entrée distale (5a) dans laquelle débouche l'extrémité proximale (2b) de l'aiguille et qui est entouré d'un tube externe de réserve d'air (6) étanche à l'air qui est clipsé et/ou collé sur l'embase, ces deux tubes communiquant l'un avec l'autre par l'extrémité proximale (5b) du tube de visualisation (5) qui n'est pas étanche à l'air. Ces deux tubes sont rigides et qui forment un ensemble rigide avec l'embase sont fabriqués dans un matériau transparent et ont une longueur de quelques centimètres. Si on le désire, l'extrémité de sortie (5b) du tube interne (5) est fermée par un bouchon (8) qui arrête le sang mais qui laisse passer l'air.

Sous l'effet de la pression sanguine le sang reflue dans le tube de visualisation interne (5) sans atteindre son extrémité proximale (5b) du fait de la pression de l'air comprimé dans le tube externe (7).

L'espace vide de sang (7) qui subsiste dans le tube interne entre la colonne de sang et l'extrémité proximale (5b) du tube permet de suivre le mouvement du sang sous l'effet des variations de la pression sanguine.

Dans la réalisation de la figure 2, l'embase (1) comporte un canal principal (9) à une extrémité duquel est montée l'aiguille (2) et dont l'extrémité opposée (9b) est ouverte pour permettre d'y enfiler une gaine (G) de guidage du cathéter ; une valve anti-retour (10) est placée dans ce canal tandis que le canal (9), entre l'extrémité de montage de l'aiguille (2) et la valve (10), communique par un passage latéral (11) avec un tube de visualisation transversal (5) entouré d'un tube externe (6) de réserve d'air. Les tubes (5) et (6) jouent respectivement les rôles des tubes (5) et (6) de la réalisation précédente.

Comme dans le cas précédent, un bouchon étanche au sang mais non étanche à l'air peut être placé à l'extrémité du tube interne (5) qui débouche dans le tube externe.

L'invention n'est pas limitée à ces exemples de réalisation.

## Revendications

1. Embase (1) d'aiguille (2) de ponction artérielle, qui comporte une chambre de visualisation tubulaire et transparente (5) accessible par une extrémité d'entrée (5a) au reflux de sang dans l'aiguille sous l'effet de la pression artérielle, des moyens (6) étant prévus pour créer à une extrémité de sortie (5b) de la chambre de visualisation une contre-pression suffisante pour maintenir à l'extrémité de sortie (5b) une colonne d'air (7) dont le volume varie sous l'effet des variations de la pression du sang dans l'aiguille, lesdits moyens comprenant une chambre de réserve d'air (6) étanche vis à vis de l'air ambiant, située à l'extérieur de la chambre de visualisation et en communication d'air avec l'extrémité de sortie (5b) de la chambre de visualisation, **caractérisée en ce que** les deux chambres forment un ensemble rigide avec l'embase et **en ce que** les volumes des chambres sont prédéterminés pour que le sang n'atteigne pas la chambre (6) de réserve d'air lorsque la pression systolique est maximale.

2. Embase selon la revendication 1 dont la chambre de réserve d'air (6) est placée autour de la chambre de visualisation (5).

3. Embase selon l'une des revendications 1 et 2 et dans laquelle l'extrémité de sortie de la chambre de visualisation (5b) est fermée par un bouchon (8) étanche au sang mais perméable à l'air.

4. Embase selon l'une des revendications 1 à 3 dont la chambre de visualisation (5) est placée dans l'axe de l'aiguille (2).

5. Embase selon l'une des revendications 1 à 3 dont la chambre de visualisation (5) est placée transversalement à la direction de l'aiguille (2).

6. Embase selon la revendication 5 et qui comporte un canal (9) qui communique par une extrémité avec l'aiguille (2) et dont l'extrémité opposée est ouverte, une valve anti-retour (10) étant placée dans ledit canal et la chambre de visualisation (5) débouchant dans le canal entre la valve anti-retour et l'extrémité du canal qui communique avec l'aiguille.

## Patentansprüche

1. Halter (1) für eine Nadel (2) zur arteriellen Punktion, der folgendes umfaßt: eine transparente und röhrenförmige Anzeigekammer (5), die über ein Eingangsende (5a) für den unter der Wirkung des arteriellen Drucks auftretenden Rückfluß des Bluts in der Nadel zugänglich ist, Mittel (6), die dafür vorgesehen sind an einem Ausgangsende (5b) der Anzeigekammer einen Gegendruck zu erzeugen, der ausreichend ist, um am Ausgangsende (5b) eine Luftsäule (7) aufrechtzuerhalten, deren Volumen unter der Wirkung der Blutdruckschwankungen in der Nadel variiert, wobei diese Mittel eine Reserveluftkammer (6) umfassen, die gegenüber der Umgebungsluft dicht ist, die außerhalb der Anzeigekammer angeordnet ist und die im Luftaustausch mit dem Ausgangsende (5b) der Anzeigekammer steht, **dadurch gekennzeichnet, daß** die zwei Kammern mit dem Halter eine starre Einheit bilden und dadurch, daß die Volumen der Kammern so festgelegt sind, daß das Blut die Reserveluftkammer (6) nicht erreicht, wenn der systolische Druck maximal ist.

2. Halter nach Anspruch 1, dessen Reserveluftkammer (6) um die Anzeigekammer (5) herum angeordnet ist.

3. Halter nach einem der Ansprüche 1 und 2 und bei dem das Ausgangsende der Anzeigekammer (5b) durch einen Stopfen (8) verschlossen ist, der für Blut dicht aber für Luft durchlässig ist.

4. Halter nach einem der Ansprüche 1 bis 3, dessen Anzeigekammer (5) in der Achse der Nadel (2) angeordnet ist.

5. Halter nach einem der Ansprüche 1 bis 3, dessen Anzeigekammer (5) quer zur Richtung der Nadel (2) angeordnet ist.

6. Halter nach Anspruch 5, der einen Kanal (9), der über ein Ende mit der Nadel (2) kommuniziert und dessen entgegengesetztes Ende offen ist, und ein Rücklaufsperrventil (10), das in dem Kanal angeordnet ist, umfaßt und wobei die Anzeigekammer (5) zwischen dem Rücklaufsperrventil und dem Ende des Kanals, das mit der Nadel kommuniziert, in den Kanal mündet.

## Claims

1. Hub (1) of an arterial puncture needle (2), comprising a tubular and transparent viewing chamber (5) accessible, via an inlet end (5a), to the reflux of blood in the needle under the effect of the arterial pressure, means (6) being provided to create, at an outlet end (5b) of the viewing chamber, a counter-pressure which is sufficient to maintain, at the outlet end (5b), an air column (7) whose volume varies under the effect of the variations in the pressure of the blood in the needle, said means comprising an air reserve chamber (6) which is leaktight to ambient air, is situated outside the viewing chamber and is in air communication with the outlet end (5b) of the viewing chamber, **characterized in that** the two chambers form a rigid assembly with the hub, and **in that** the volumes of the chambers are predetermined such that the blood does not reach the air reserve chamber (6) when the systolic pressure is maximal.

2. Hub according to Claim 1, in which the air reserve chamber (6) is disposed around the viewing chamber (5) .

3. Hub according to one of Claims 1 and 2, and in which the outlet end of the viewing chamber (5b) is closed by a plug (8) which is leaktight to blood but permeable to air.

4. Hub according to one of Claims 1 to 3, in which the viewing chamber (5) is disposed in the axis of the needle (2).

5. Hub according to one of Claims 1 to 3, in which the viewing chamber (5) is disposed transversely with respect to the direction of the needle (2).

6. Hub according to Claim 5, and comprising a channel (9) which communicates at one end with the needle (2) and whose opposite end is open, a nonreturn valve (10) being disposed in said channel, and the viewing chamber (5) opening into the channel between the nonreturn valve and the end of the channel communicating with the needle.
